# EUROPÄISCHE PATENTANMELDUNG

(11) **EP 1 364 664 A1**
(43) Veröffentlichungstag der Anmeldung: **26.11.2003**
(21) Anmeldenummer: 03090138.3
(22) Anmeldetag: 13.05.2003
(51) Int. Cl.: A61L 31/10, A61F 2/06

(54) **Verfahren zum Beschichten eines Stents mit einer Polysaccharid-Lage sowie dazugehörige Stents**

(30) Priorität: 24.05.2002 DE 10223310
(71) Anmelder: BIOTRONIK Mess- und Therapiegeräte GmbH & Co Ingenieurbüro Berlin, 12359 Berlin (DE)
(72) Erfinder: Bayer, Gerd, 91054 Erlangen (DE); Nagel, Markus, 91301 Forchheim (DE); Borck, Alexander, 38106 Braunschweig (DE)
(74) Vertreter: Eisenführ, Speiser & Partner

(57) **Zusammenfassung**

Die Erfindung betrifft Verfahren zum Beschichten von Stents sowie nach diesem Verfahren hergestellte Stents. Die Erfindung liegt die Aufgabe zugrunde Verfahren zur Beschichtung von Stents mit einer Polysaccharid-Lage anzugeben, die ein verbessertes Haftvermögen auf der Substratoberfläche des Implantats besitzt und in der Bereitstellung entsprechend funktionalisierter Stents. Dies wird u.a. durch kovalentes Anbinden einer nicht-quervernetzten Hyaluronsäure an eine Substratoberfläche des Stents unter Bildung einer Hyaluronsäure-Lage und Quervernetzung der Hyaluronsäure-Lage erreicht.

## Beschreibung

Die Erfindung betrifft Verfahren zum Beschichten von Stents, insbesondere von kardiovaskulären Implantaten, mit einer Polysaccharid-Lage bzw. Polysaccharid-Derivat-Lage sowie nach diesen Verfahren hergestellte Stents.

Zum Hintergrund der Erfindung ist festzuhalten, dass Polysaccharide als biokompatibel bekannt sind. Typische Vertreter sind in diesem Zusammenhang Heparin, Chitosan, Alginat oder Hyaluronsäure. Letztere haben sich zum einen als sehr körperverträglich erwiesen, zum anderen sind Beschichtungen aus Hyaluronsäure hydrophil und folglich die damit versehenen Geräte gut implantierbar.

Mit Polysacchariden im allgemeinen und Hyaluronsäure im speziellen beschichtete Implantate und Verfahren zu deren Beschichtung mit Hyaluronsäure sind aus dem Stand der Technik zahlreich bekannt. So offenbart die US 6,042,876 A einen Führungsdraht für lmplantierungszwecke, der mit einem solchen hydrophilen Polysaccharid, wie Hyaluronsäure oder Chondroitinsulfat beschichtet ist.

Die US-A-4,957,744 bezieht sich auf vernetzte Ester von Hyaluronsäure, die für verschiedenste medizinische und kosmetische Artikel sowie pharmazeutische Zusammensetzungen verwendet werden. Die vernetzten Ester resultieren aus der Veresterung von mehrwertigen Alkoholen mit zwei oder mehr Carboxy-Gruppen der Hyaluronsäure. Solche vernetzten Ester sind besonders auf dem Gebiet bioresorbierbarer Kunststoffe für medizinische und chirurgische Artikel verwendbar.

Schließlich bezieht sich die WO 8802623 A1 auf Biomaterialien mit biokompatibler Oberfläche, wobei unter einer Vielzahl von Ausgangsmaterialien und Bindungsmechanismen unter anderem die Verwendung von Hyaluronsäure zur Herstellung einer biokompatiblen Kontaktlinse offenbart wird.

Sofern die vorgenannten Druckschriften Beschichtungsverfahren für medizinische Gerätschaften und insbesondere Stents betreffen, weisen diese den Nachteil auf, dass die erzielten Polysaccharid-Lagen keine ausreichenden Haftfestigkeiten auf der Substratoberfläche erzielen.

Die Aufgabe der vorliegenden Erfindung besteht demnach in der Angabe eines Verfahrens zur Beschichtung von Stents mit einer Polysaccharid-Lage, die ein verbessertes Haftvermögen auf der Substratoberfläche des Implantats besitzt und in der Bereitstellung entsprechend funktionalisierter Stents.

Diese Aufgabe wird durch die alternativen Verfahren mit den Merkmalen der Ansprüche 1, 2, 3, 4 oder 5 sowie den dazugehörigen Stent nach Anspruch 6 gelöst. Laut Anspruch 1 wird die Aufgabe durch folgende kennzeichnende Verfahrensschritte gelöst:
- kovalentes Anbinden einer nicht-quervernetzten Hyaluronsäure an die Substratoberfläche des Stents unter Bildung der Polysaccharid-Lage und
- Quervernetzung der Hyaluronsäure -Lage (Variante l).

In einer alternativen Ausgestaltung des erfindungsgemäßen Verfahrens gemäß nebengeordnetem Anspruch 2 wird statt der Quervernetzung der aufgebrachten nicht-quervernetzten Hyaluronsäure-Lage eine weitere Lage einer quervernetzten Hyaluronsäure auf die erste nicht-quervernetzte Hyaluronsäure-Lage aufgebracht (Variante II).

Nach einer dritten erfindungsgemäßen Variante (III) wird das Verfahren wie folgt durchgeführt:
- kovalentes Anbinden einer nicht-quervernetzten Hyaluronsäure an eine Substratoberfläche des Stents unter Bildung einer ersten Hyaluronsäure-Lage,
- kovalentes Anbinden einer zweiten nicht-vernetzten Lage aus Hyaluronsäure und
- Quervernetzung der zweiten Hyaluronsäure-Lage.

Eine vierte Variante (IV) sieht vor, dass die Verfahrensschritte wie folgt durchzuführen sind:
- Anbinden einer Haftvermittlerschicht an eine Substratoberfläche des Stents,
- kovalentes Anbinden einer nicht-quervernetzten Hyaluronsäure an die Haftvermittlerschicht unter Bildung einer Hyaluronsäure-Lage und
- Quervernetzung der Hyaluronsäure-Lage.

Schließlich erfolgt nach einer fünften Variante (V) die Beschichtung in folgender Weise:
- Anbinden einer Haftvermittlerschicht an eine Substratoberfläche des Stents,
- kovalentes Anbinden einer nicht-quervernetzten ersten Lage aus Chitosan an die Haftvermittlerschicht unter Bildung einer Chitosan-Lage und
- Aufbringung einer zweiten Lage aus quervernetzter oder nichtquervernetzter Hyaluronsäure.

Die Grundvarianten l und V der erfindungsgemäßen Verfahren sorgen durch die kovalente Anbindung des nicht-quervernetzten Polysaccharids für eine signifikante Erhöhung der Haftfähigkeit der Polysaccharid-Lage, was experimentell nachweisbar ist. Dabei kann die weitere Lage als nichtquervernetztes Polysaccharid aufgebracht und anschließend quervernetzt oder unmittelbar als quervernetztes Polysaccharid aufgebracht werden.

Weitere Vorteile insbesondere der Variante II liegen in dem primären Aufbringen einer gleichmäßigen Polysaccharidschicht und Ankopplung einer sekundären, vorzugsweise dickeren Lage, die im Gegensatz zu anderen Hydrogelfilmen vergleichbarer Dicke ein geringes Quellvermögen besitzt. Aufgrund ihrer physikalischen und chemischen Eigenschaften eignen sich Polysaccharidschichten, wie Hydrogelfilme bzw. Polymermatrizen, für die Einbettung von Wirkstoffen, um mittels lokaler Wirkstofffreisetzung die biokompatible Wirkung zu erhöhen bzw. lokal eine pharmakologische Wirkung zu erzielen. Im Vergleich zu üblichen Hydrogelfilmen bzw. Polymermatrizen weisen Polysaccharidschichten aus Glykosaminoglykanen, insbesondere aus Hyaluronsäure, zusätzlich eine eigene pharmakologische Wirkung auf.

Ein besonders geeignetes Polysaccharid zur Anwendung in dem erfindungsgemäßen Verfahren ist die bereits angesprochene Hyaluronsäure, die auf verschiedenste Substratoberflächen von Implantaten aufgebracht werden kann. Alloplastische Gefäßwandstützen - sogenannte "Stents" - sind üblicherweise mit amorphen Siliziumkarbid (a-SiC:H) beschichtet, das mit Hyaluronsäure eine besonders innige und haftfeste Verbindung eingeht.

Schließlich können funktionelle Beschichtungen durch das abwechselnde Aufbringen von jeweils mehreren Lagen von nicht-quervernetzten und quervernetzten Polysacchariden erzielt werden.

Weitere Merkmale, Einzelheiten und Vorteile der Erfindung ergeben sich aus den folgenden Ausführungsbeispielen:

Das erfindungsgemäße Verfahren wird anhand der Beschichtung einer Substratoberfläche aus amorphem Siliziumkarbid beschrieben, das beispielsweise auf einem Stent mit einem Grundgerüst aus einer Tantal-Legierung aufgebracht ist. Grundzüge der Aktivierung der Siliziumkarbid-Substratoberfläche sind dabei der DE 195 33682 A1 der Anmelderin entnehmbar, die die Anlagerung und Immobilisierung von Heparin auf einer Siliziumkarbidbeschichtung offenbart.

### I. Anbindung der Polysaccharide

### Beispiel 1 - Anbindung über ein Benzophenonderivat

Demnach wird die Substratoberfläche mit Wasser gespült und in einer 20 x 10⁻⁶-molaren Fmoc-p-Bz-Phe-OH-Lösung in N,N'-Dimethylformamid (DMF) inkubiert. Die als photoaktive Spacer-Substanz wirksame Fmoc-p-Bz-Phe-OH-Lösung ist als handelsübliches Produkt "Fmoc-p-Bz-Phe-OH", Produktnummer B 2220 der Firma "Bachem Biochemica GmbH", Heidelberg zu beziehen. Die Reduktion des Benzophenons wird durch die Bestrahlung mit UV-Licht eingeleitet. Nach der UV-Bestrahlung wird die Reaktionslösung abgegossen und die Substratoberfläche mehrmals mit destilliertem Wasser gespült.

Der nächste Schritt umfasst die Abspaltung der Fmoc-Schutzgruppe mit 25%-iger Piperidinlösung in DMF. An der nun freiliegenden und reaktionsfähigen Aminogruppe erfolgt die Bindung der Hyaluron-Säure. Dazu wird erst nicht-quervernetzte Hyaluron-Säure kovalent an die so behandelte Substratoberfläche angebunden. Anschließend kann die so gebildete Polysaccharid-Lage quervernetzt werden.

Alternativ zur vorher beschriebenen photochemischen Reaktion lassen sich Polysaccharide und insbesondere Hyaluronsäure nasschemisch kovalent mit silanisierten Benzophenonen, Epoxysilanen und Aminosilanen als Spacer-Substanzen kovalent an die Substratoberfläche, insbesondere an die Siliziumkarbid-Substratoberfläche binden.

### Beispiel 2 - Anbindung über silanisiertes Benzophenonderivat

Die nasschemische kovalente Anbindung eines silanisierten Benzophenons, insbesondere von 4-(3'-Chlorodimethylsilyl)propyloxybenzophenon, erfolgt nasschemisch im organischen Lösungsmittel wie Toluol bei Raumtemperatur über Nacht in Anwesenheit von Et₃N als Katalysator. Nach der Inkubationszeit werden die Substrate in Chloroform und anschließend in Methanol gespült. Danach wird das Schichtsystem Substrat-Spacer mit einer 0,l%-2%igen wässrigen Hyaluronsäurelösung benetzt und anschließend getrocknet. Die kovalente Anbindung der Hyaluronsäure an das vorliegende Benzophenon erfolgt unter Einwirkung von UV-Strahlung bei einer Wellenlänge von 340 nm, welche die Reduktion des Benzophenons einleitet.

Alternativ kann die photochemische Reaktion auch in wässriger Hyaluronsäurelösung durchgeführt werden. Diese photochemische Reaktion führt zu einer kovalenten Bindung zwischen dem Benzophenon und einer C-H-Gruppe der Polymerkette, insbesondere der Hyaluronsäure. Diese an die Substratoberfläche kovalent angebundene Polysaccharidlage kann anschließend quervernetzt werden.

### Beispiel 3 - Anbindung über Epoxysilane

Für die nasschemische Beschichtung von Siliziumkarbid-Substraten mit Epoxysilanen werden die Substrate zuerst gereinigt und anschließend für eine Stunde bei einer Temperatur von 75°C getrocknet. Die Silanisierung der warmen Substrate mit (3-(2,3-Epoxypropoxy)-propyl)-trimethoxysilan erfolgt unter Immersion im organischen Lösungsmittel. Anschließend werden die silanisierten Substrate getrocknet und im organischen Lösungsmittel gewaschen. Die anschließende kovalente Anbindung der Hyaluronsäure erfolgt im wässrigen Lösungsmittel über Nacht unter Schütteln bei Raumtemperatur. Alternativ kann die Anbindung von Hyaluronsäure durch Inkubation in einer 0,25%igen Hyaluronsäure-Lösung in einer 0,1m HCI bei 65°C für 1 h erfolgen. Chitosan kann durch Inkubation in einer 0,2%igen Chitosan-Lösung in einer 1-2%igen Essigsäurelösung bei 65°C für 1 h gebunden werden. Die Stents werden anschließend mit deionisiertem Wasser gespült und danach getrocknet. Diese an die Substratoberfläche kovalent angebunden Polysaccharidlage kann anschließend quervernetzt werden.

### Beispiel 4 - Anbindung von Chitosan

Durch eine kovalent angebundene Chitosan-Lage (Monolage) lässt sich eine gute Haftvermittlung erreichen. Das Glykosaminoglykan Chitosan (Mw: 100.000 bis 1.000.000 Dalton) wird in einer chemischen Mehrstufenreaktion mit Hilfe eines Spacers kovalent an eine amorphe Siliziumkarbid-Schicht (a-SiC:H), die den Grundkörper des Stents bedeckt, angebunden.

Im ersten Schritt des Beschichtungsprozesses wird der Spacer - die photoaktive Benzophenonverbindung Fmoc-p-BZ-Phe-OH (N-(9-Fmoc)-l-(4-Benzoyl)-phenylalanin; 2 ml; 10 mmol/l; erhältlich bei der Fa. Bachem) - im Lösungsmittel N,N'-Dimethylformamid (DMF) durch photochemische Reaktion kovalent an das Siliziumkarbid angebunden. Nach Spülen mit DMF erfolgt das Abspalten der Fmoc-Schutzgruppe des Spacers mit einer 20 %igen Piperidinlösung in DMF. Anschließend liegt die Aminogruppe des Spacers frei vor.

Der Stent wird dann in einer 0,2%igen Lösung von Chitosan in 1%iger Essigsäure und N-(3-Dimethylaminopropyl)-N'-ethylcarbodiimidhydrochlorid (50 mg/ml) für mind. 12 Stunden in Eiswasser inkubiert. Die kovalente Ankopplung des Chitosans erfolgt durch Knüpfung einer Peptidbindung zwischen der aktivierten Carbonsäure des Spacers und der Aminogruppe des Chitosans oder Bildung einer Esterbindung zwischen der aktivierten Carbonsäure des Spacers und der Hydroxylgruppe des Chitosans. Nach Reaktionsende wird die Probe mehrfach mit deionisiertem Wasser gespült und anschließend getrocknet.

### Beispiel 5 - Anbindung an plasmaabgeschiedenes Polymer

Im Folgenden wird ein Anwendungsbeispiel für das Aufbringen einer Haftvermittlungsschicht näher erläutert. An die gereinigte Substratoberfläche wird eine wenige Nanometer dicke Polymerschicht aufgebracht, welche als Haftvermittler dient und für eine anschließende kovalente Anbindung einer Polysaccharid-Lage geeignete funktionelle Gruppen an der Oberfläche aufweist. Eine solche Haftvermittlerschicht lässt sich durch eine Plasmapolymerisation von N-Heptylamnin und Acetaldehyd erreichen.

Die Plasmapolymerisation erfolgt mit einer 40 kHz Plasmaanlage Modell Piko der Firma Diener electronics. Alternativ zu den genannten Precourser können Acetaldehyd, Amylalkohol, Allylamin, Acetessigsäureethylester oder Acrylsäure verwendet werden. Die plasmapolymerisierten Schichten weisen aufgrund ihrer Hydrophilie eine gute Benetzung mit Hyaluronsäurelösungen auf. Weiterhin lassen sich an den funktionellen Oberflächen eine dünne Schicht aus Hyaluronsäure oder anderen Polysacchariden mit Hilfe von Glutaraldehyd, Epichlorhydrin, oder Carbodiimiden ankoppeln.

Der Rezipient wird zum endgültigen Entfernen von Restgas mit Sauerstoff gespült, dabei wird kontinuierlich evakuiert. Es wird ein Spülgasfluss von ca. 40 cm3/min. eingestellt. Der Probenraum wird für 10 min. gespült und für die Oberflächenaktivierung wird in Anwesenheit eines Teflonblocks das Plasma gezündet. Die Leistung des Reaktors beträgt ca. 200 W und während der Oberflächenbehandlung wird ein Sauerstoffstrom von ca. 40 cm³/min. aufrecht erhalten. Die Aktivierung und gleichzeitige Plasmareinigung dauert 5-10 min.

Nach erfolgter Aktivierung bzw. Reinigung wird die Leistung auf 80 W runtergeregelt und der Precourser wird in den Rezipienten eingelassen. Die Polymerisationsdauer beträgt bei geöffneten Aerometer 5 min. Nach erfolgter Abschaltung kann eine weitere Oberflächenaktivierung mit Sauerstoff erfolgen, jedoch beträgt dann die Leistung nur 80 W bei einer Dauer von ca. 30 sek. Diese kurze Oberflächenaktivierung führt zu einer weiter verbesserten Benetzbarkeit der Oberflächen.

An die abgeschiedene Haftvermittlerschicht aus dem N-Heptylaminplasmapolymer wird anschließend die Hyaluronsäure mit Hilfe eines wasserlöslichen Carbodiimids kovalent an den Komplex Substrat-Haftvermittlerschicht angebunden. Die kovalente Anbindung der Hyaluronsäure an das Acetaldehydplasmapolymer erfolgt direkt mit Hilfe eines Diimidazols oder unter Anbindung einer Polyethyleniminzwischenlage, welche mittels reduktiven Aminierung aufgebracht wird. Die kovalente Anbindung der Hyaluronsäure an diesen Substrat-Haftvermittler-Komplex erfolgt mit Hilfe eines wasserlöslichen Carbodiimids. Diese an die Haftvermittlerschicht kovalent angebunden Polysaccharid-Lage kann anschließend quervernetzt werden.

### Beispiel 6 - Anbindung über derivatisierte Polyhdroxybuttersäure

Alternativ zum Verfahren der Plasmapolymeristation kann die Substratoberfläche durch derivatisierte Polyhydroxybuttersäure, welche eine experimentell nachgewiesene gute Schichthaftung auf Siliziumkarbid und Metallen aufweist, funktionalisiert werden. Die Funktionalisierung der Polyhydroxybuttersäure erfolgt durch Aminierung. Die kovalente Anbindung der Hyaluronsäure an die Aminogruppe der funktionalisierten Polyhydroxybuttersäure (Haftvermittlerschicht) erfolgt mit Hilfe eines wasserlöslichen Carbodiimids unter Ausbildung einer Peptidbindung.

### Beispiel 7 - Anbindung von Chitosan über ein Aminosilan

Eine Monolage aus Chitosan kann wie folgt erzeugt werden:

Der vorgereinigte Stent wird bei 75°C für 30 Minuten im Trockenofen getrocknet. Anschließend wird der noch warme Stent für 10 Minuten in einer Silanlösung aus 3 ml wasserfreies Toluol, 20 µl 3-[2-(2-Aminoethylamino)ethylamino]propyl-trimethoxysilan und 70 µl Et₃N bei Raumtemperatur unter mehrmaligem leichtem Schütteln inkubiert. Es folgt Trocknung der Stents bei 75° C für 1 Stunde. Danach wird der Stent mit Toluol oder Chloroform gespült und erneut getrocknet. Es folgt eine kovalente Ankopplung von Adipinsäure über eine Lösung von 10mg/ml Adipinsäure in Wasser zur Erzeugung funktioneller Carbonylfunktionen an der Oberfläche des Implantats. Die Adipinsäure wird zuvor in THF oder DMF mit einem Carbodiimid oder Diimidazol aktiviert. Nach Spülen im deionisiertem Wasser und Trocknung folgt die Anbindung von Chitosan. Dazu wird das Implantat in eine 0,2%ige Lösung von Chitosan in einer 1-2%igen Essigsäurelösung bei Raumtemperatur für 1-4 Stunden inkubiert. Es folgt Spülen mit deionisiertem Wasser und Trocknung.

### II. Vernetzungs- und Beschichtungsverfahren

Im Folgenden werden nun Vernetzungs- und Beschichtungsverfahren von Hyaluronsäure auf Implantatoberflächen näher erläutert. Die beschriebenen Verfahren eignen sich dabei um
- eine an das Substrat kovalent angebundene nicht quervernetzte Polysaccharid-Lage zu vernetzen,
- ein unvernetztes Polysaccharid an eine quervernetzte oder unvernetzte Polysaccharid-Lage kovalent anzubinden, bzw.
- ein quervernetztes Polysaccharid an eine quervernetzte oder unvernetzte Polysaccharid-Lage kovalent anzubinden.

### Beispiel 8 - Vernetzung mit Glutaraldehyd

Die Vernetzung von Hyaluronsäure mit Glutaraldehyd ist wie folgt realisierbar:

Das Implantat wird mit einer 0,1-2%igen Hyaluronsäurelösung beschichtet und anschließend einer Vernetzerlösung für mehrere Stunden ausgesetzt. Die Vernetzerlösung besteht aus 240 ml Aceton, 80 ml Glutaraldehyd in 25%ige Lösung in Wasser und 1,6 ml HCL 3 M. Danach wird die Vernetzerlösung gegen eine neue Lösung ausgetauscht und es wird wieder bei Raumtemperatur für mehrere Stunden inkubiert. Die mittels Glutaraldehyd vernetzte Hyaluronsäure wird mehrfach in destilliertem Wasser gewaschen. Anschließend wird die Probe in einer 0,5-3%igen Lösung des Natriumcyanoborhydrids für eine 1 Stunde bei Raumtemperatur inkubiert. Die Fixiererlösung wird abgezogen und es folgen mehrere Waschschritte in bidestilliertem Wasser und isotonischer Kochsalzlösung.

Eine Vernetzung der Hyaluronsäure mit bifunktionellen Aldehyden und Formaldehyd erfolgt in einem Verfahren analog zur Vernetzung der Hyaluronsäure mit Glutaraldehyd.

### Beispiel 9 - Vernetzung mit Epichlorhydrin

Zur Vernetzung von Hyaluronsäure durch Epichlorhydrin werden 0,38 g Hyaluronsäure in 90 ml Wasser gelöst. Zu der Lösung werden 10 g NaOH und 6,8 ml wässrige Amoniaklösung (25%) gegeben. Die Reaktionslösung wird auf 20°C temperiert. Nach Erreichen der Temperatur werden 19,6 ml Epichlorhydrin dazugegeben. Die Lösung wird bei 20°C für 24 Std., gerührt. Anschließend wird die vernetzte Hyaluronsäure gegen bidestiliertes Wasser dialysiert. Die verwendeten Dialyseschläuche besitzen eine Ausschlussgrenze von 120.000 DA.

### Beispiel 10 - Vernetzung mit Divinylsulfon

Zur Vernetzung von Hyaluronsäure durch Divinylsulfon werden 2 g Hyaluronsäure in 50 ml einer 0,1 m wässrigen NaOH Lösung unter Erhalt einer 2%igen Lösung gelöst. Die Lösung wird auf Eis gestellt. Bei erfolgtem Temperaturausgleich werden 2 ml Divinylsulfon zugegeben. Die entstehende Zweiphasenmischung wird für 15 Min. auf Eis gerührt. Nach 5 Minuten ist nur noch eine Phase zu beobachten. Die Implantate werden in diese Lösung getaucht und anschließend getrocknet.

### Beispiel 11- Vernetzung mit Ethylenalykol-diclycidylether

Zur Vernetzung von Hyaluronsäure mit Ethylenglykol-diclycidylether wird eine 0,1- 2%ige Hyaluronsäurelösung in einer 0,9%igen isotonischen Kochsalzlösung hergestellt. Die Reaktion wird bei 25°C durchgeführt. Als Vernetzer werden bis zu 10 Mol% Ethylenglykol-diclycidylether bezogen auf die Repetiereinheit der Hyaluronsäure zugegeben.

### Beispiel 12-Vernetzung mit Diimidazol

Ferner ist eine Vernetzung und kovalente Anbindung der Hyaluronsäure an Schichtsysteme aus amorphem Siliziumkarbid-Spacer und einem amorphen Siliziumkarbid-Spacer-Polysaccharidmonolayer mit Diimidazol realisierbar. Das Implantat mit angebundenem Spacer bzw. mit einer Polysaccharid-Lage wird in eine Diimidazol-haltige Acetonlösung getaucht. Der Substrat-Spacer-Komplex bzw. die Polysaccharid-Lage wird für mindestens 30 Minuten in der Diimidazol-haltigen Acetonlösung aktiviert und anschließend in eine wässrige Hyaluronsäurelösung getaucht bzw. mit einer Hyaluronsäurelösung besprüht. Zur Sprühbeschichtung wird der Stent für 0,5-1 sek. angesprüht bei einem Druck der Trägerluft von 2-4 bar. Zwischen den Sprühschritten wird der Stent 15-30 sek. unter Warmluftzufuhr getrocknet. Durch Wiederholung der Schritte lässt sich ein gewünschter Schichtaufbau auf den Stent erreichen. Um ein Schichtwachstum zu erreichen, wird dieser Prozess mehrfach wiederholt.

### Beispiel 13 - Vernetzung mit Säuredichloriden oder Phosphoroxidchlorid

Die Vernetzung der OH- und NHR-Gruppen von Polysacchariden erfolgt mit Hilfe von Säurendichloriden bzw. Phosphoroxidchlorid unter Ausbildung von Ester- oder Amidgruppen und unter Freisetzung von HCL im organischen Lösungsmittel.

### lll. Derivatisierung der Polysaccharid-Lage

Eine Derivatisierung des beschichteten Hyaluronsäure-Hydrogels auf dem Implantat ist ebenfalls wie folgt realisierbar.

### Beispiel 14 - Sulfatierung

Durch eine polymeranaloge Umsetzung von Hyaluronsäure z.B. mittels eines SO₃*Pyridin-Komplexes wird der enzymatische Abbau der Hyaluronsäure in vivo verzögert bzw. die Hyaluronsäure im Körper stabilisiert, wie folgendes Anwendungsbeispiel zeigt:

Unter Stickstoffatmosphäre wird in einem thermostatisierbaren Doppelwandreaktor mit Rückflusskühler und Rührer die Hyaluronsäure in trockenem Pyridin suspendiert. Zu dieser Suspension gibt man einen Schwefeltrioxid-Pyridin-Komplex und erwärmt auf die gewünschte Reaktionstemperatur. Nach 3 Std. wird die Reaktion abgebrochen und die auf Raumtemperatur abgekühlte Suspension in die fünffache Menge Methanol gegossen. Das ausgefallene Polymer wird abfiltriert, in Wasser gelöst und gegen entionisiertes Wasser dialysiert. Da das Produkt teilweise als Pyridiniumsalz vorliegt und die Polymerketten untereinander intermolekular verestert sind, wird der pH-Wert der Polymerlösung nach der Dialyse durch Zugabe von 0,1 N Natronlauge auf 11 eingestellt. Dialyse und Titration werden dreimal wiederholt. Bei einem pH-Wert von 7,3 wird das Polymer gefriergetrocknet.

Eine Variation des Sulfatierungsgrades bei dieser polymeranalogen Umsetzung ist durch die Menge an zugesetztem Sulfatierungsreagenz SO₃*Pyridin, die Reaktionszeit und die Reaktionstemperatur möglich.

### IV. Einbettung von Pharmaka

Eine Wirkstoffbeladung mit geeigneten Pharmaka erfolgt in der Regel nach Vernetzung und Fixierung der Polysaccharidschicht im gequollenen Zustand. Alternativ kann der Wirkstoff mit Hilfe eines Sprüh- oder Tauchverfahrens vor der Beschichtung oder während der Beschichtung mit dem Polysaccharid erfolgen. Die Wirkstoffeinbettung erfolgt in der Regel über Diffusionsprozesse.

### Beispiel 15 - Einbettung von Cyclosaporin

Der Wirkstoff wird über ein Tauchverfahren in eine Hyaluronsäure-Lage, wie sie nach einem der vorhergehenden Beispiele erhältlich ist, eingebettet. Dazu wird das Implantat in eine Lösung von 15 mg Cyclosporin pro ml eines paritätischen Ethanol-Wasser-Gemisches getaucht. Das Verhältnis von 1:1 von Ethanol zu Wasser hat sich für die Durchführung des Diffusionsprozesses als überraschend effektiv erwiesen. Andere Verhältnisse, insbesondere mit erhöhtem Ethanolgehalt, verlangsamen die Einbettung. Das Implantat verbleibt je nach Schichtdicke und Vernetzungsgrad der Polysaccharidlage für zumindest eine Stunde in der Lösung. Anschließend wird das Implantat entnommen und getrocknet. Bei einer Beschichtungsmenge von 0,5 mg Hyaluronsäure beträgt die auf diese Weise einlagerbare Cyclosporinmenge mindestens 0,2 mg.

## Patentansprüche

1. Verfahren zum Beschichten eines Stents mit einer Polysaccharid-Lage mit folgenden Verfahrensschritten:
- kovalentes Anbinden einer nicht-quervernetzten Hyaluronsäure an eine Substratoberfläche des Stents unter Bildung einer Hyaluronsäure-Lage und
- Quervernetzung der Hyaluronsäure-Lage.

2. Verfahren zum Beschichten eines Stents mit einer Polysaccharid-Lage mit folgenden Verfahrensschritten:
- kovalentes Anbinden einer nicht-quervernetzten Hyaluronsäure an eine Substratoberfläche des Stents unter Bildung einer ersten Hyaluronsäure-Lage und
- kovalentes Anbinden einer weiteren Lage einer quervernetzten Hyaluronsäure auf die erste Hyaluronsäure-Lage.

3. Verfahren zum Beschichten eines Stents mit einer Polysaccharid-Lage mit folgenden Verfahrensschritten:
- kovalentes Anbinden einer nicht-quervernetzten Hyaluronsäure an eine Substratoberfläche des Stents unter Bildung einer ersten Hyaluronsäure-Lage,
- kovalentes Anbinden einer zweiten nicht-quervernetzten Lage aus Hyaluronsäure und
- Quervernetzung der zweiten Hyaluronsäure-Lage.

4. Verfahren zum Beschichten eines Stents mit einer Polysaccharid-Lage mit folgenden Verfahrensschritten:
- Anbinden einer Haftvermittlerschicht an eine Substratoberfläche des Stents,
- kovalentes Anbinden einer nicht-quervernetzten Hyaluronsäure an die Haftvermittlerschicht unter Bildung einer Hyaluronsäure-Lage und
- Quervernetzung der Hyaluronsäure-Lage.

5. Verfahren zum Beschichten eines Stents mit einer Polysaccharid-Lage mit folgenden Verfahrensschritten:
- Anbinden einer Haftvermittlerschicht an eine Substratoberfläche des Stents,
- kovalentes Anbinden einer nicht-quervernetzten ersten Lage aus Chitosan an die Haftvermittlerschicht unter Bildung einer Chitosan-Lage und
- Aufbringung einer zweiten Lage aus quervernetzter oder nichtquervernetzter Hyaluronsäure.

6. Stent mit einer Beschichtung aus einer Polysaccharid-Lage nach einem der Ansprüche 1 bis 5.
